(19) **Europäisches Patentamt**
**European Patent Office**
**Office européen des brevets**

(11) Veröffentlichungsnummer: **0 223 190**
**B1**

(12) # EUROPÄISCHE PATENTSCHRIFT

(45) Veröffentlichungstag der Patentschrift: **23.05.90**

(51) Int. Cl.⁵: **G 01 N 1/10, G 01 N 1/20**

(21) Anmeldenummer: **86115659.4**

(22) Anmeldetag: **11.11.86**

(54) **Verfahren zur Gewinnung von Flüssigkeitsproben, insbesondere Milchproben.**

(30) Priorität: **16.11.85 DE 3540769**

(43) Veröffentlichungstag der Anmeldung:
**27.05.87 Patentblatt 87/22**

(45) Bekanntmachung des Hinweises auf die
Patenterteilung:
**23.05.90 Patentblatt 90/21**

(84) Benannte Vertragsstaaten:
**AT CH DE ES FR LI**

(56) Entgegenhaltungen:
**EP-A-0 098 966**
**EP-A-0 161 552**
**DE-B-1 933 754**
**DE-B-2 245 487**
**DE-C-1 236 960**
**DE-U-8 414 249**
**FR-A-1 363 478**

(73) Patentinhaber: **OTTO TUCHENHAGEN GmbH & Co. KG**
**Berliner Strasse 10 Postfach 1140**
**D-2059 Büchen (DE)**

(72) Erfinder: **Mieth, Hans Otto, Dipl.-Ing.**
**Sandkrug 3**
**D-2058 Schnakenbek/Elbe (DE)**

(74) Vertreter: **Glaeser, Joachim, Dipl.-Ing.**
**Patentanwälte DIEHL GLAESER HILTL & PARTNER Königstrasse 28**
**D-8000 Hamburg 50 (DE)**

Courier Press, Leamington Spa, England.

EP 0 223 190 B1

# EP 0 223 190 B1

**Beschreibung**

Die Erfindung betrifft ein Verfahren zur Gewinnung von Flüssigkeitsproben, insbesondere Milchproben, aus einer von einem Anlieferungsbehälter zu einem Sammelbehälter führenden Förderleitung nach dem Oberbegriff des unabhängigen Patentanspruches 1.

Eine Vorrichtung zur Realisierung eines derartigen Verfahrens zur Probeentnahme von Milch ist beispielsweise aus dem DE—GM 84 14 249 bekannt.

Weitere Vorrichtungen zur Übernahme von Milch, bei denen die Probe mit annähernd gleichen Mitteln wie bei der vorgenannten Vorrichtung gewonnen wird, sind in der DE—PS 12 36 960 und in der DE—AS 22 45 487 beschrieben.

Während bei der Vorrichtung gemäß DE—PS 12 36 960 die Probeentnahme aus der Förderleitung über eine Zweigleitung mit einem unveränderlichen Entnahmequerschnitt erfolgt, ist bei der Vorrichtung gemäß DE—AS 22 45 487 in der Förderleitung vor dem Luftabscheider eine Entnahmeeinrichtung angeordnet, welche mit Hilfe eines Handgriffes beispielsweise in zwei Stellungen von unterschiedlichem Entnahmequerschnitt gebracht und mit Hilfe einer Verriegelungseinrichtung in den Stellungen verriegelt werden kann. Die Entnahmeeinrichtung hat die Aufgabe eines Mengenteilers, mit Hilfe dessen ein dem zu überführenden Gesamtvolumen proportionales Volumen in das Probenvorlaufgefäß abgezweigt werden kann. Oberstes Ziel einer jeglichen Probeentnahme dieser Art ist es, im Probenvorlaufgefäß ein dem zu überführenden Gesamtvolumen möglichst repräsentatives Volumen zu stapeln, so daß im Anschluß daran aus diesem Volumen ein relativ kleines Probevolumen in ein Probengefäß abgeführt werden kann.

Die Repräsentativität des im Probenvorlaufgefäß gesammelten Milchvolumens wird durch den sogenannten Verschleppungseinfluß beeinträchtigt. Unter Verschleppung versteht man die Vermischung des im Probenvorlaufgefäß gesammelten Milchvolumens mit Milchresten vom vorangegangenen Lieferanten, die im Annahmesystem als Restmilch- oder Haftmilchvolumen verblieben sind und über den Mengenteiler in das Probenvorlaufgefäß gelangten.

Das sogenannte Restmilchvolumen im Annahmesystem vor dem Mengenteiler läßt sich durch bestimmte verfahrenstechnische Maßnahmen stark reduzieren. Der verbleibende Teil wird jedoch immer, in einem streng repräsentativ arbeitenden Annahme-system, bei der Messung erfaßt und anteilig, entsprechend der Mengenteilereinstellung, in das Probenvorlaufgefäß überführt.

Die sich bei zwei aufeinanderfolgenden Lieferanten aus dem Restmilchvolumen ergebende Differenz der Keimkonzentrationen (hier sei die Keimzahl als besonders verschleppungsrelevante Einflußgröße ausgewählt) ist, wie eine Keimbilanz leicht zeigt, in erster Näherung ausschließlich abhängig von dem zu überführenden Gesamtvolumen und wird proportional zu dessen Kehrwert reduziert. Das Mengenteilerverhältnis bleibt deshalb ohne Einfluß auf diesen Teil des Verschleppungsfehlers; es ist daher gleichgültig, ob ein großes oder ein kleines Volumen in das Probenvorlaufgefäß abgezweigt wird, und ob letzteres weniger oder mehr befüllt ist.

Anders verhält es sich mit dem sogenannten Haftmilchvolumen im Probenvorlaufgefäß. Hier liegt es auf der Hand, daß dessen Einfluß um so geringer ist, je mehr Milch in das Probenvorlaufgefäß abgezweigt wurde und somit zu einer Verdünnung des Haftmilchvolumens des vorangegangenen Lieferanten beitragen kann. Dieser Tatsache tragen Anordnungen zur Probeentnahme von Milch der einleitend beschriebenen Art Rechnung, die einen Mengenteiler aufweisen, bei dem mehrere Teilungsverhältnisse einstellbar sind. Jede Mengenteilereinstellung ist einem bestimmten zu überführenden Volumebereich zugeordnet, wobei das System so bemessen ist, daß am Ende eines Volumenbereiches das Probenvorlaufgefäß nahezu vollständig befüllt ist. In diesem Fall ist dann eine maximale Verdünnung des Haftmilchvolumens innerhalb des Probenvorlaufgefäßes gegeben. Probleme ergeben sich allerdings bei der Umstellung des Mengenteilers auf den nächsten Volumenbereich. Wird beispielsweise gerade das Mindestvolumen eines Volumenbereiches angenommen, dann ist das Probenvorlaufgefäß über die jeweils zugeordnete Stellung des Mengenteilers minimal gefüllt. In diesem Falle ergeben sich die ungünstigsten Bedingungen fur eine Verdünnung des im Probenvorlaufgefäß befindlichen Haftvolumens.

Die Reduzierung des Einflusses des vorstehend genannten Haftvolumens — und nur diese Reduzierung — auf den Verschleppungsfehler ist Gegenstand der aus dem GM 84 14 249 bekannten Probeentnahmevorrichtung. Diese ermöglicht das Abzweigen der Milch in zwei Zeitabschnitten mit einem dem jeweiligen Zeitabschnitt zugeordneten Proportionalitätsfaktor, wobei in der ersten Phase der Proportionalitätsfaktor, bezogen auf das insgesamt umzufüllende Milchvolumen, so groß ist, daß am Ende dieser Phase ein vorgegebenes erstes Teilvolumen erreicht ist, das dann bis auf ein einem wesentlich kleineren Proportionalitätsfaktor entsprechendes Restvolumen ausgeschieden wird, um in der anschließenden zweiten Phase die Abzweigung des Teilvolumens mit diesem wesentlich kleineren Proportionalitätsfaktor und die Entnahme der Probe nach Mischen des Restvolumens und des in der zweiten Phase abgezweigten Teilvolumens durchzuführen.

Ob die vorgeschlagenen Maßnahmen, die einen nicht unerheblichen apparativen Aufwand erfordern, gerechtfertigt sind, hängt davon ab, ob sie eine signifikante Reduzierung des Verschleppungsfehlers bewirken. Dieses kann aber nur, wie einleitend erörtert, im Zusammenhang mit dem Restmilchvolumen im System vor dem Mengenteiler beurteilt werden. Die Praxis hat jedenfalls gezeigt, daß der Einfluß des Haftvolumens, bezogen auf den Einfluß des Restvolumens, in der Regel vernachlässigbar gering ist, so daß durch die vorgeschlagenen Maßnahmen die Repräsentativität der Probe nicht verbessert wird.

2

Wenn einerseits die Wirksamkeit der vorstehend beschriebenen Probeentnahmevorrichtung im Hinblick auf eine signifikante Reduzierung des Verschleppungsfehlers in Zweifel zu ziehen ist, so weist diese Vorrichtung eine andere Besonderheit auf, die allerdings in der formulierten Aufgabenstellung nicht explizit herausgestellt ist.

Die Probeentnahmevorrichtung bedarf nämlich keiner Voreinstellung durch die Bedienungsperson, da nur ein Annahme-Mengenbereich zwischen minimalem und maximalen Annahmevolumen gegeben ist. Dem vorgenannten Vorteil stehen allerdings eine Reihe von Nachteilen gegenüber:

1. Die Probeentnahmevorrichtung sieht eine Rückführung der Milchmenge aus dem Sammelgefäß in die Ansaugleitung vor. Damit ergibt sich eine Reduzierung der Ansaugleistung des Systems und ein Zeitverlust, insbesondere bei Gesamtvolumina, die geringfügig oberhalb des Mindestannahmevolumens leigen.

2. Durch die Rückführung ist eine Meßwertverfälschung gegeben, da von der rückgeführten Milch ein Zweites Mal Probe genommen wird. Diese Maßnahme ist um so kritischer zu bewerten, als die rückgeführte Milch in hohem Maße durch Verschleppung verfälscht ist.

3. Durch die Notwendigkeit eines nieveaugesteuerten Umschaltens des Mengenteilers von einem Entnahmequerschnitt, der einen relativ großen Proportionalitätsfaktor realisiert, auf einen mit einem wesentlich kleineren Proportionalitätsfaktor strömt zwängsläufig Milch am Mengenteiler vorbei, die entweder überhaupt nicht oder überproportional erfaßt wird. Dieser Umstand wirkt sich insbesondere dann stark verfälschend aus, wenn das zu überführende Gesamtvolumen geringfügig oberhalb des Mindestannahmevolumens liegt. Dadurch wird bei diesen Annahmerverhältnissen die Repräsentativität der Probenahme erheblich beeinträchtigt, da gerade bei den in Frage kommenden kleineren Anlieferungsbehältern am Ende der Milchübernahme stark aufgerahmte und mit anderen Milchinhaltsstoffen aufkonzentrierte Milch vorliegt.

Ausgehend vom vorgenannten Stand der Technik liegt der Erfindung die Aufgabe zugrunde, ein Verfahren zu schaffen, mit dem bei höchstmöglicher Repräsentativität der Probenentnahme der aus dem Haftmilchvolumen im Probenvorlaufgefäß resultierende Verschleppungsfehler auf ein Mindestmaß reduziert wird, bei dem die zu überführende Milch einmal am Mengenteiler vorbeigeführt und geteilt wird, bei dem keinerlei Einstell- und/oder Anpassungsmaßnahmen im Vorwege oder im Zuge des Vefahrensablaufes durch eine Bedienungsperson erforderlich sind, und bei dem die Abzweigung eines Teilstromes aus dem zu überführenden Flüssigkeitsvolumen fortlaufend und mit einem sich stetig ändernden Proportionatitätsfaktor erfolgt.

Diese Aufgabe wird erfindungsgemäß durch ein Verfahren nach den Merkmalen des unabhängigen Patentanspruches gelöst.

Das Probenvorlaufgefäß verhält sich in diesem zweiten Zeitabschnitt wie ein kontinuierlich betriebener Rührkessel, dem über eine Zulaufleitung ein bestimmter Volumenstrom mit einer bestimmten Konzentration zugeführt und dem über eine Ablaufleitung ein mengenmäßig entsprechender Volumenstrom mit einer sich durch das Verweilzeitverhalten ergebenden Ablaufkonzentration entnommen wird. Eine unstetige, sprunghafte Änderung des Proportionalitätsfaktors wird dadurch erfindungsgemäß vermieden.

Auf das vorgenannte Probenvorlaufgefäß finden die wissenschaftlich gesicherten Erkenntnisse eines kontinuierlich betriebenen Idealkessels (KIK) Anwendung (vergl. Winnacker/Küchler, Chemische Technologie I, 4. Auflage, Abschnitt 4, insbes. Seiten 274—287). Der stetig aus der Förderleitung abgezweigte Volumenstrom ist dabei so zu bemessen, daß im Probenvorlaufgefäß unter Berücksichtigung der über den Ablauf des Probenvorlaufgefäßes ausgetragenen Ausgangskonzentration stets die für das übernommene Volumen repräsentative Konzentration im Anlieferungsbehälter abgebildet wird.

Die notwendigen theoretischen Überlegungen zur rechnerischen Lösung des anstehenden Problems werden nachfolgend erläutert. Ziel dieser Überlegungen ist die Ermittlung eines volumenabhängigen Teilungsverhältnisses bzw. einer Teilungsfunktion, mit dem bzw. mit der zu jedem Zeitpunkt der Milchübernahme der in das Probenvorlaufgefäß abzuzweigende Volumenstrom zu bestimmen ist.

Das Verfahren zeigt unter anderem auf, daß entweder der Zulauf oder der Ablauf des Probenvorlaufgefäßes regelbar ist. Für die letztgenannte Lösung spricht im Hinblick auf die praktische Realisierbarkeit die Erkenntnis, daß ein der Zulaufleitung zum Probenvorlaufgefäß vorgeschaltetes, steuerbares Pitotrohr schwieriger als eine in der Ablaufleitung angeordnete Einrichtung zur Steuerung des Durchsatzes realisierbar ist.

Eine andere Ausgestaltung des Verfahrens sieht vor, daß ein in einem festen Teilungsverhältnis zum Volumenstrom in der Förderleitung stehender Volumenstrom aus dieser abgezweigt und in das Probenvorlaufgefäß überführt wird, wobei das Volumen des Probenvorlaufgefäßes stetig und abhangig vom überführten Volumen vorgrößerbar ist. Der Vorteil dieser Verfahrensvariante besteht darin, daß auf eine Reduzierung des abgezweigten Volumenstromes auf nicht unproblematisch kleine Werte verzichtet werden kann, und dafür eine nach den ermittelten Gesetzmäßigkeiten vorzunehmende Volumenvergrößerung des Probenvorlaufgefäßes nach einer Vergrößerungsfunktion zu erfolgen hat.

Eine andere Ausgestaltung des Verfahrens vermeidet die Abzweigung kleinster Volumenströme dadurch, daß die Teilungsfunktion C(V) bzw. die Vergrößerungsfunktion M(V) durch impulsweise Ansteuerung der letztere realisierenden Vorkehrungen dargestellt wird.

Da zu jedem beliebigen Zeitpunkt der übernahme des Volumens die für das übernommene Volumen repräsentative Konzentration im Anlieferungsbehälter im Probenvorlaufgefäß abzubilden ist, bedarf das Verfahren zu jedem Zeitpunkt der Kenntnis des bis zu diesem Zeitpunkt übernommenen Volumens.

Die Messung dieses Volumens kann, wie dies eine andere Ausgestaltung des erfindungsgemäßen Verfahrens vorsieht, in einem, einem Luftabscheider vorgeschalteten, Abschnitt der Förderleitung erfolgen.

Das Verfahren vereinfacht sich, wenn nach einer anderen Ausgestaltung des erfindungsgemäßen Verfahrens die Messung über den ohnehin vorhandenen Volumenzähler erfolgt, der dem Luftabscheider nachgeschaltet ist. Diese Maßnahme setzt allerdings voraus, daß der Luftsabcheider im Rahmen seines Schaltspiels ein im Verhältnis zu der Meßgenauigkeit des vorgeschlagenen Verfahrens vernachlässigbares Speicherverhalten aufweist.

Die Erfindung wird nachstehend an Hand der Zeichnungen beispielsweise erläutert.

Es zeigen

Figur 1 eine Anordnung in schematischer Darstellung zur Durchführung des Verfahrens gemäß der Erfindung, wobei das Teilungsverhältnis über eine Steuerung des Zulaufs des Probenvorlaufgefäßes einstellbar ist;

Figur 2 eine andere Anordnung in schematischer Darstellung zur Durchführung des Verfahrens gemäß der Erfindung, wobei das Teilungsverhältnis über eine Steuerung des Ablaufs des Probenvorlaufgefäßes einstellbar ist;

Figur 3 eine weitere Anordnung in schematischer Darstellung zur Durchführung des Verfahrens gemäß der Erfindung, wobei das Volumen des Probenvorlaufgefäßes stetig und abhängig vom überführten Volumen vegrößerbar ist und

Figur 4 eine Skizze zur Erläuterung der Modellbildung für die rechnerische Ermittlung des Teilungsverhältnisses nach dem Verfahren gemäß der Erfindung.

Die dem Verfahren gemäß der Erfindung zugrunde liegenden theoretischen Überlegungen sollen zunächst an Hand der Figur 4 erläutert werden.

Der Verfahrensablauf sieht vor, daß in einer ersten Überführungsphase mit einem konstanten Teilungsverhältnis $C=C_1$ das Probenvorlaufgefäß mit seinem Volumen $V^*$ vollständig befüllt wird. Diese Befüllung sei mit der Übernahme eines Volumens $V=V_1$ abgeschlossen. Im Probenvorlaufgefäß befindet sich nunmehr ein dem überführten Volumen $V_1$ repräsentatives Teilvolumen, das die Konzentration $k_{1m}(V_1)=k_1$ aufweist.

In einer sich der ersten Überführungsphase anschließenden zweiten Überführungsphase soll nunmehr ein Volumen $\Delta V$ mit einer Konzentration $k_2$, die sich beispielsweise von der Konzentration $k_1$

des Volumens $V_1$ unterscheidet, überführt werden. Mit Beginn der Übernahme des Volumens $\Delta V$ stellt sich die Frage, wie das Teilungsverhältnis $C(V)$ zu verändern ist, damit am Ende der Übernahme des Volumens $\Delta V$ mit seiner Konzentration $k_2$ im Probenvorlaufgefäß ein dem Gesamtvolumen $V_1+\Delta V$ repräsentatives Teilvolumen vorliegt. Die repräsentative Keimzahl für $V_1+\Delta V$ beträgt

$$K_{2m}=(k_1V_1+k_2V_2)/(V_1+\Delta V) \qquad (1)$$

Das Probenvorlaufgefäß verhält sich für ein zu übernehmendes Volumen $V > V_1$ wie ein technischer Raktor, dem man in der chemischen Technologie als kontinuierlich betriebenen Rührkessel bezeichnet. Im Rahmen dieser Betrachtung sei ideales Rührkesselverhalten (KIK-Verhalten) angenommen, das heißt, das zugeführte Teilvolumen $\Delta V.C(V)$ mit seiner Konzentration $k_2$ wird mit dem im Probenvorlaufgefäß befindlichen Volumen $V^*$ ideal vermischt.

Unter der Bedingung $V^* =$ konstant muß ein entsprechendes Teilvolumen $\Delta V \, C(V)$ mit der zeitlich veränderlichen Konzentration

$$k(t)=k_1+\Delta k(t) \qquad (2)$$

den Ablauf des Probenvorlaufgefäßes verlassen. Am Ende der Übernahme des Volumens $\Delta V$ mit der Konzentration $k_2$ zum Teitpunkt $t_e$ muß im Ablauf und damit auch im gesamten Probenvorlaufgefäß die Konzentration

$$k(t_e)=k_{2m} \qquad (3)$$

vorliegen.

Der Idealkessel zeichnet sich dadurch aus, daß die Konzentration seines Inhaltes an jedem Ort und zu jeder Zeit immer der Konzentration im Ablauf entspricht.

Aus der Theorie des kontinuierlich betriebenen Idealkessels (KIK-Prinzip) weiß man, daß bei konstantem Volumendurchsatz und einer Konzentrationsänderung am Eintritt um $\Delta k_o$ von $k_1$ auf $k_2$, am Austritt eine nach einer e-Funktion sich einstellende Konzentrationsänderung

$$k(t)=\Delta k_o(1-\exp(-t)(\tau)) \qquad (4)$$

EP 0 223 190 B1

vorliegt.

Das Volumen $\Delta V$ ist bei konstantem Volumenstrom $Q$ in der Zeit

$$t=te-ta=\Delta V/Q \qquad (5)$$

uberführt.

Die Zeit $\tau$ bedeutet die mittlere Verweilzeit im Probenvorlaufgefäß. Sie beträgt ($Q_v$ sei der Volumenstrom zum Probenvorlaufgefäß)

$$\tau=V^*/Q_v, \qquad (6)$$

wobei

$$Q_v=\Delta V\, C(V)/\Delta t \qquad (6a)$$

ist.

Die vorstehende Beziehung gilt für konstante mittlere Verweilzeit $\tau$ und damit für $Q_v$ = konstant.
Der realistische Ansatz geht aber davon aus, daß sich $Q_v$ und damit über

$$Q_v=C(V).Q \qquad (7)$$

auch das Teilungsverhältnis $C(V)$ stetig ändert.

Zur Vereinfachung wird im zu übernehmenden Volumenbereich $\Delta V$ näherungsweise mit einem mittleren Teilungsverhältnis $C(V)=C_m(V)$ gerechnet.

Mit den Gleichungen 1 bis 7 erhält man für das mittlere Teilungsverhältnis im Volumenbereich $\Delta V$

$$C_m(V)=V^*/V(1n(1+\Delta V/V_1)). \qquad (8)$$

Für den Grenzfall $\Delta V \rightarrow o$ geht Gleichung (8) über in

$$C_m(V)=V^*/V_1=C_1 \qquad (9)$$

Trägt man den realistischen Verhältnissen Rechnung, daß das Teilungsverhältnis $C(V) \neq$ konstant während der Übernahme des Teilvolumens $\Delta V$ ist, so ändert dieses nichts daran, daß auch $C(V)$, ebenso wie $C_m(V)$, stets größer ist als $C^*(V)$:

$$C_m(V),\, C(V) > C^*(V), \qquad (10)$$

wobei

$$C^*(V)=V^*/V \text{ ist} \qquad (10a)$$

Das Teilungsverhältnis $C^*(V)$ gilt für eine fiktive, praktisch nicht durchführbare, Probennahme, bei der dieses Verhältnis über das gesamte anzunehmende Volumen $V$ exakt immer (in genauer Vorkenntnis des zu übernehmenden Gesamtvolumens) so eingestellt ist, daß am Ende der übernahme des Gesamtvolumens das Probenvorlaufgefäß mit seinem Volumen $V^*$ gerade vollständig gefüllt ist.

Da beim kontinierlich betriebenen Idealkessel (KIK) zu jedem Zeitpunkt ein Volumenstrom das Probenvorlaufgefäß verläßt, dessen Konzentration nach Maßgabe der vorliegenden Verweilseitverteilung beeinflußt wird von der Eintrittskonzentration des zulaufenden Volumenstromes (Überspüleffekt), ist es verständlich, wenn hier das Teilungsverhältnis größer ist als jenes ohne diesen Überspüleffekt.

Der Idealkessel idealisiert die Verhältnisse. Tatsächlich kann es zu Kurzschlüssen oder aber zu unvollständiger Durchmischung im Probenvorlaufgefäß kommen. Das reale Verhalten eines konkreten Probenvorlaufgefäßes ist aber in einer einmaligen experimentellen Untersuchung erfaßbar. Sein Verweilzeitverhalten (W(t) für die Summenkurve) ist mit bekannten Methoden zu ermitteln (vgl. Winnacker/Küchler, 4. Aufl., Abschn. 4.3). Daher wird im konkreten Fall die theoretisch ermittelte Teilungsfunktion $C(V)$ bzw. $C_m(V)$ experimentell anzupassen sein.

Nachdem de theoretische Ansatz zur Ermittlung der Teilungsfunktion bzw. des Teilungsverhaltnisses $C(V)$ in dem zweiten Zeitabschnitt des vorgeschlagenen Verfahrens erläutert wurde, soll nunmehr das gesamte Verfahren mit seinen Varianten im Zusammenhang beschrieben werden.

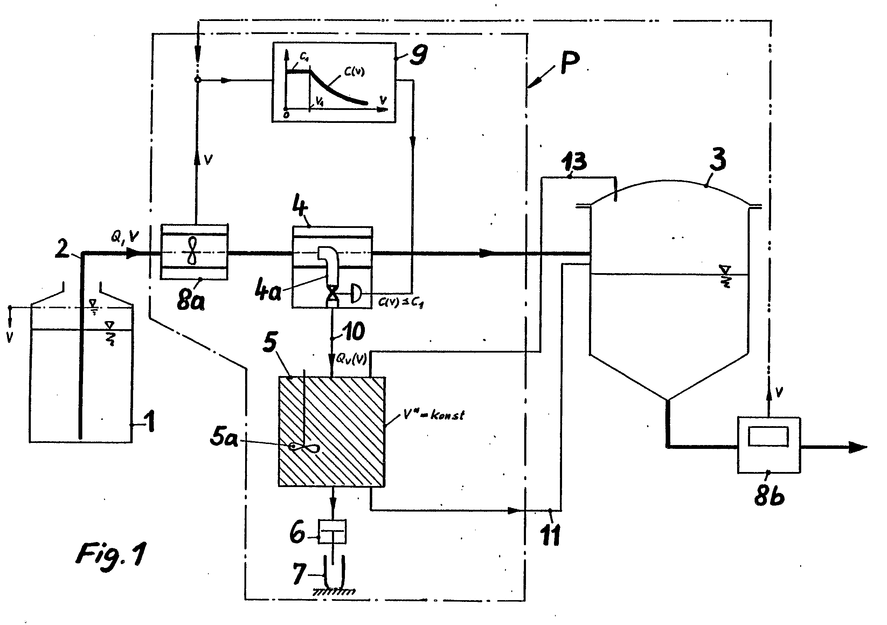

In eine Förderleitung 2 (Figur 1), die von einem Anlieferungsbehälter 1 zu einem nicht dargestellten Sammelbehälter führt, ist ein Luftabscheider 3 zwischengeschaltet. Vor letzterem befindet sich in der Förderleitung 2 eine Entnahmevorrichtung 4 (Mengen- oder Volumenteiler), der beispielsweise ein in die Förderleitung 2 eingreifendes, steuerbares Pitotrohr aufweist. Dieses Pitotrohr 4a ist einer Zulaufleitung 10, die von der Förderleitung 2 abzweigt un zu einem Probenvorlaufgefäß 5 führt, vorgeschaltet. Eine Ablaufleitung 11 verbindet die Unterseite des Probenvorlaufgefäßes 5 mit dem Laufabscheider 3. Über eine Druckausgleichsleitung 13 wird der Druck im Kopfraum des Probenvorlaufgefäßes 5 jenem im Luftabscheider 3 gleichgemacht, Dadurch ist eine vom Staudruck der Flüssigkeitsströmung in der

5

Förderleitung 2 abhängige Probenentnahme über das steuerbare Pitotrohr 4a möglich. Innerhalb des Probenvorlaufgefäßes 5 befindet sich eine Rühreinrichtung 5a. Weiterhin ist im unteren Bereich des Probenvorlaufgefäßes 5 eine Probenmenge-Abgabeneinrichtung 6 vorgesehen, mit der eine definierte Probenmenge in ein Probengefäß 7 abführbar ist.

Über eine dem Luftabscheider 3 nachgeschaltete Volumenmeßeinrichtung 8b ist das über die Förderleitung 2 in den nicht dargestellten Sammelbehälter überführte Gesamtvolumen meßtechnisch erfaßbar.

Die insoweit beschriebene Anordnung ist Stand der Technik. Das vorgeschlagene Verfahren gemäß der Erfindung zeichnet sich nun insbesondere dadurch aus, daß eine über die nachgeschaltete Volumenmeßeinrichtung 8b oder eine der Entnahmevorrichtung 4 vorgeschaltete Volumenmeßeinrichtung 8a zu jedem Zeitpunkt einer Milchübernahme eine Volumeninformation über das bis zu diesem Zeitpunkt überführte Teilvolumen liefert. Diese Volumeninformation wird einer Stelleinrichtung 9 zugeführt, in der die Teilungsfunktion bzw. das Teilungsverhaltnis C(V) sowohl für den ersten als auch für den nachgeschalteten zweiten Zeitabschnitt enthalten ist. Diese Teilungsfunktion C(V) läßt sich theoretisch und/oder experimentell gewinnen. In dem dargestellten Ausführungsbeispiel bewirkt die Stelleinrichtung 9 eine Steuerung des steuerbaren Pitotrohres 4a, wodurch der über die Zulaufleitung 10 zum Probenvorlaufgefäß 5 fließende Volumenstrom $Q_v$ veränderbar ist. Das Probenvorlaufgefäß 5 weist ein konstantes Volumen $V^*$ auf.

In dem ersten Zeitabschnitt wird das Probenvorlaufgefäß 5 über die Entnahmevorrichtung 4 und einem konstanten Teilungsverhältnis $C_1$ vollständig befüllt. Am Ende des ersten Zeitabschnittes wurde ein Teilvolumen $V_1$ aus dem Anlieferungsbehälter 1 übernommen, wobei $V_1—V^*$ in das Probenvorlaufgefäß überführt wurden (Ende des ersten Zeitabschnittes). Bei der weiteren Überführung von Teilvolumina aus dem Anlieferungsbehälter 1 in den Sammelbehälter erhält die Stelleinrichtung 9 entweder über die nachgeschaltete oder über die vorgeschaltete Volumenmeßeinrichtung 8b bzw. 8a diesbezügliche Volumeninformationen, so daß über die in der Stelleinrichtung 9 niedergelegte Teilungsvunktion C(V) ein variabler, stetig kleiner werdender Volumenstrom $Q_{v(V)}$ in das Probenvorlaufgefäß 5 überführt wird. Dieser Volumenstrom ist über die Teilungsfunktion so bemessen, daß die für das übernommene Volumen V repräsentative Konzentration im Anlieferungsbehälter 1 zu jedem Zeitpunkt im Probenvorlaufgefäß 5 abgebildet ist. Mit dem im mit P gekennzeichneten Bereich enthaltenen Probenentnahmesystem ist eine Variante des Verfahrens gemäß der Erfindung realisierbar (Figur 1). Es handelt sich um jene Verfahrenvariante, bei der die Teilungsfunktion bzw. das Teilungsverhältnis C(V) über eine Steuerung des Zulaufes des Probenvorlaufgefäßes 5 einstellbar ist.

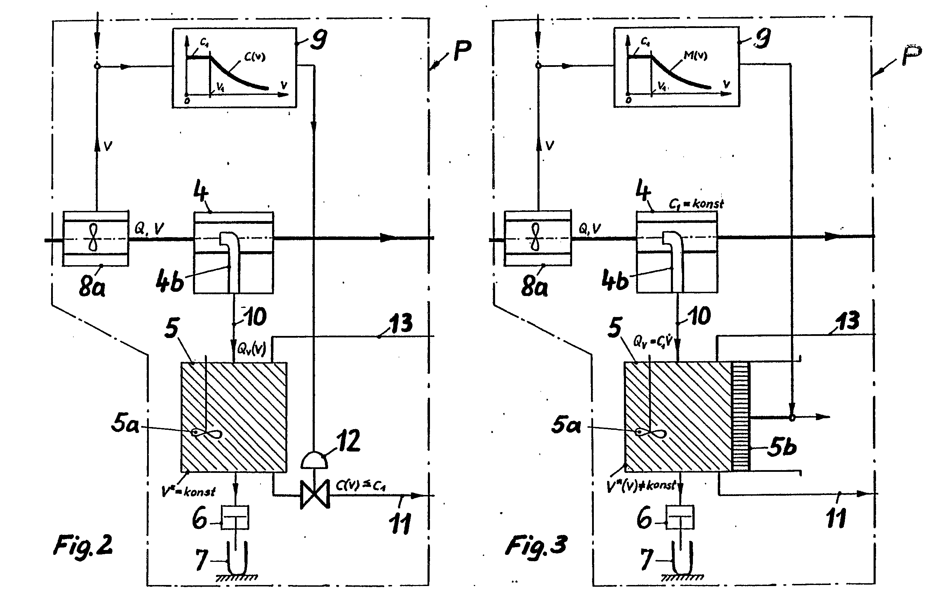

In Figur 2 ist eine weitere Verfahrensvariante dargestellt, bei der die Teilungsfunktion C(V) über eine Steuerung des Ablaufes des Probenvorlaufgefäßes 5 erfolgt. Die Entnahmevorrichtung 4 beinhaltet in diesem Falle ein festeingestelltes Pitotrohr 4b, während in der Ablaufleitung eine Steuereinrichtung 12, beispielsweise eine Einrichtung mit steuerbarem Durchsatz, vorgesehen ist. Auf die Steuereinrichtung 12 hat der Stelleinrichtung 9 nach Maßgabe der dort vorliegenden Teilungsfunktion C(V) Zugriff. Die übrige Anordnung zur Durchführung des Verfahrens und der Verfahrensablauf selbst wurden bereits unter Figur 1 beschrieben.

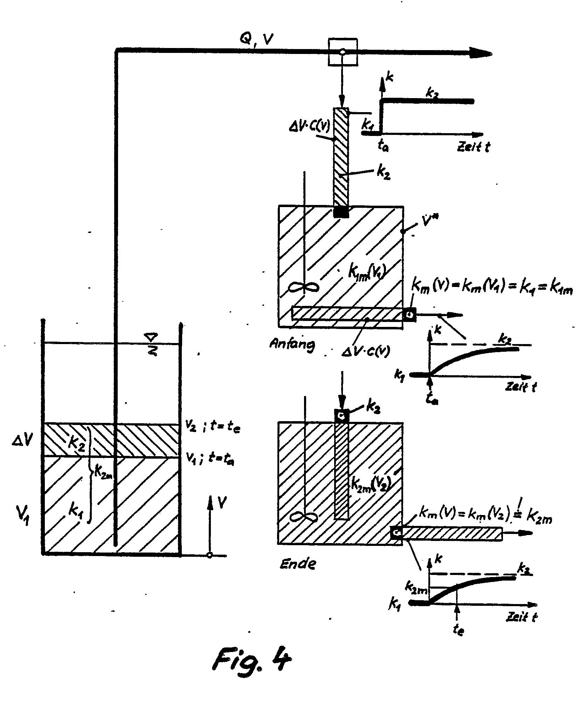

Die Verfahrensvariante nach der Anordnung gemäß Figur 3 trägt der Erkenntnis Rechnung, daß es gleichgültig ist, ob ein variabler, stetig kleiner werdender Volumenstrom $Q_v$ in ein Probenvorlaufgefäß 5 mit konstantem Volumen $V^*$ oder ob alternativ ein in einem festen Teilungsverhältnis zum Volumenstrom Q in der Förderleitung stehender Volumenstrom $Q_v$ aus dieser abgezweigt und in ein nach der Vegrößerungsfunktion M(V) variables, stetig größer werdendes Probenvorlaufgefäß 5 überführt wird. Die variable Gestaltung des Volumens des Probenvorlaufgefäßes 5 ist im dargestellten Ausführungsbeispiel schematisch durch eine verschiebbare Gefäßwand 5b angedeutet.

Zur Realisierung dieses gegenständlichen Merkmals bieten sich eine Vielzahl von konstruktiven Möglichkeiten, die allerdings nicht Bestandteil des Verfahrens gemäß der Erfindung sein können. Es sei nur an dieser Stelle angedeutet, daß eine Veränderung des Volumens des Probenvorlaufgefäßes 5 beispielsweise durch eine steuerbare Überlaufhöhe im Behälter erreicht werden kann. Die übrige Anordnung zur Durchfuhrung des Verfahrens gemäß der Erfindung bleibt unverändert; enbenso der Verfahrensablauf. Es gilt hier sinngemäß die Beschreibung zu Figur 1. Der Vorteil dieser Verfahrensvariante besteht auch darin, daß der Probenentnahmequerschnitt nicht reduziert werden muß. Zur Frage der Querschnittreduzierung wird nachfolgend Stellung genommen.

Das im Probenvorlaufgefäß gestapelte und nach dem KIK-Prinzip behandelte Teilvolumen muß nicht, wie vorstehend beschrieben, in vom Staudruck abhängiger Weise über ein Pitotrohr gewonnen werden, wobei entweder der Zu- oder der Ablauf des Probenvorlaufgefäßes gesteuert wird. Es ist ebenso möglich, das Teilvolumen, abhängig vom jeweils überführten Volumen, zwangsweise über eine in der Zulaufoder Ablaufleitung des Probenvorlaufgefäßes angeordnete, steuerbare Fördereinrichtung (beispielsweise Kreisel- oder Verdeängerpumpe im weitesten Sinne) aus der Förderleitung abzuzweigen und in das Probenvorlaufgefäß zu überführen (Umkehrung des volumenstromabhängigen Zudosierens).

Die Probenentnahme, beispielsweise in vom Staudruck abhängiger Weise über ein Pitotrohr durchgeführt, erfordert nicht nur beim Erfindungsgegenstand, sondern auch bei bekannten Probeentnahmesystemen, bei größen zu überführenden Milchmengen am Ende des Übernahmevorganges (beim Gegenstand gemäß GM 84 14 249 bereits nach dem ersten Zeitabschnitt) eine relativ kleine Probenentnahmeöffnung, die hinsichtlich Verstopfung und Reinigung problematisch sein kann. Außerdem ist eine, wie vorstehend gefordert stetige Querschnittsveränderung mit einem gewissen Konstruktionsaufwand verbunden.

Der erfindungsgemäße Verfahren schafft hier mit einer vorteilhaften Verfahrensvariante Abhilfe, indem der zum Probenvorlaufgefäß abgezweigte Volumenstrom $Q_V$ impulsweise gesteuert wird. Der Probenentnahmequerschnitt ist für maximalen Volumenstrom $Q_V$ ausgelegt. Die Volumenstromreduzierung im Abhängigkeit von der Teilungsfunktion C(V) erfolgt nicht durch Querschnittsreduzierung, sondern durch impulsweise, d.h. getaktete Freigabe des vollen Querschnittes. Hier bieten sich im Prinzip zwei gleichwertige Varianten an:

1. bei konstanter Impulsbreite $d_o$ (Öffnung des Probenentnahmequerschnittes über eine immer gleiche Zeitspanne) erfolgt die Anpassung des Volumenstromes $Q_V$ nach Maßgabe der Teilungsfunktion C(V) durch Veränderung der Impulsfrequenz f (f=1/T), wobei T der zeitliche Abstand benachbarter geöffneter Probenentnahmequerschnitte ist;

2. bei konstanter Impulsfrequenz $f_o$ erfolgt die Anpassung des Volumenstromes $Q_V$ nach Maßgabe der Teilungsfunktion C(V) durch Veränderung der Impulsbreite d.

Prinzipiell kann die Realisierung der Teilungsfunktion C(V) mittels Impulssteuerung auch auf die vorstehend beschriebenen Vergrößerungen des Probenvorlaufgefäßes angewandt werden. Dieses wird dann bei nach wie vor festem Teilungsverhältnis, d.h. konstantem Probenentnahmequerschnitt, impulsweise, sinngemäß nach der vorstehend beschriebenen Variante 1 und 2, nach einer Vergrößerungsfunktion M(V) vergrößert.

Zusammenfassend seien noch einmal die Vorteile des Verfahrens gemäß der Erfindung zusammengestellt. Das Verfahren und daraus resultierende Anordnungen zu seiner Durchführung erfordern gegenüber bekannten Verfahren und Anordnungen keinen erhöhten Aufwand. Es ist lediglich zusätzlich eine intelligente Stelleinrichtung 9 erforderlich, die heute zweckmäßig mit einer Mikroprozessorsteuerung ausgestattet ist, in der die Teilungsfunktion bzw. das Teilungsverhältnis C(V) oder die Vergrößerungsfunktion M(V) ohne Schwierigkeit realisiert werden kann. Eine Voreinstellung des Probeentnahmesystems P durch eine Bedienerperson ist nicht notwendig. Es arbeitet im ersten und zweiten Zeitabschnitt im Bereich zwischen einem Mindestannahmevolumen und einem nur durch das Fassungsvermögen des Sammelbehälters begrenzten maximalen Annahmevolumens völlig selbsttätig und autark. Das Probenvorlaufgefäß 5 wird im ersten Zeitabschnitt, falls das dazu notwendige Teilvolumen im Anlieferrungsbehälter 1 vorliegt, mit einem Grundvolumen befüllt. Dabei ergibt sich bei höchstmöglicher Repräsentativät der Probenentnahme (keine verlorene Spülung) ein aus dem Haftmilchvolumen im Probenvorlaufgefäß resultierender Verschleppungsfehler, der auf ein Mindestmaß reduziert wird. Weitere Einstell- oder Anpassungsmaßnahmen sind, wie vorstehend bereits erwähnt, im Vorwege oder im Zuge des Verfahrensablaufes durch eine Bedienungsperson nicht erforderlich.

Das Probenvorlaufgefäß 5 bleibt während des zweiten Zeitabschnittes mit dem Grundvolumen befüllt; es arbeitet wie ein kontinuierlich betriebener Idealkessel. Eine Rückführung oder zwischenzeitliche Umschaltvorgänge mit Ausschüben des im Probenvorlaufgefäß 5 gestapelten Volumens sind nicht erforderlich. Dadurch und durch den aus dem KIK-Verhalten resultierenden Überspüleffekt werden die Betriebssicherheit des Gesamtsystems und dessen Reinigungsfähigkeit im Anschluß an die Probenentnahme erhöht.

**Patentansprüche**

1. Verfahren zur Gewinnung von Flüssigkeitsproben, insbesondere Milchproben, aus einer von einem Anlieferungsbehälter zu einem Sammelbehälter führenden Förderleitung, bei dem jeweils während der Überführung eines zu prüfenden Flüssigkeitsvolumens ein dem Gesamtvolumen proportionales Volumen abgezweigt, in einem Probenvorlaufgefäß gesammelt und gemischt und bei Beendigung der Überführung in ein vom Gesamtvolumen unabhängiges Probe- und in ein Restvolumen unterteilt wird, von denen das Probevolumen in ein Probegefäß und das Restvolumen in den Sammelbehälter abgeführt werden, und bei dem das Abzweigen in das Probenvorlaufgefäß in zwei Zeitabschnitten, in denen jeweils verschiedene Teilungsverhältnisse wirksam sind, erfolgt, wobei am Ende der ersten Zeitabschnittes ein mit einem konstanten Teilungsverhältnis aus einem Teil des zu überführenden Gesamtvolumens gewonnenes Grundvolumen in das Probenvorlaufgefäß überführt ist, dadurch gekennzeichnet, daß in dem nachfolgenden zweiten Zeitabschnitt, falls noch ein weiteres Teilvolumen zur Übernahme ansteht, ein nach einer Teilungsfunktion C(V) bestimmter Volumenstrom in das Grundvolumen, das konstant gehalten wird, fortlaufend abgezweigt und mit diesem vermischt wird, daß ein dem zugeführten Volumenstrom mengenmäßig entsprechender Volumenstrom aus dem Grundvolumen fortlaufend angeführt wird, und daß sich die Teilungsfunktion C(V) zu jedem Zeitpunkt der Überführung des zu prüfenden Flüssigkeitsvolumens aus dem bis zu diesem Zeitpunkt überführten Gesamtvolumen und der zugeordneten Verweilzeitverteilungsfunktion für das als kontinuierlich betriebenen Rührkessel zu betrachtende Probenvorlaufgefäß derart bestimmt, daß zu jedem beliebigen Zeitpunkt der Übernahme des Volumens V die für dieses Volumen repräsentative Konzentration im Anlieferungsbehälter im Probenvorlaufgefäß abgebildet ist.

2. Verfahren nach Anspruch 1, dadurch gekennzeichnet, daß in dem zweiten Zeitabschnitt mit variablen, stetig kleiner werdenden Teilungsverhältnissen C(V), deren Anfangswert dem Wert aus dem ersten Zeitabschnitt entspricht und die vom bis zum Zeitpunkt t überführten Volumen

$$V(t) = \int_0^t Q(t)dt$$

abhängig sind, ein Volumenstrom $Q_v(V)$ in das Probenvorlaufgefäß überführt wird, wobei sich die Teilungsfunktion C(V), abhängig von der Verweilzeitverteilungsfunktion $W(t)=1-\exp(-t/\tau)$ für das näherungsweise als kontinuierlich betriebenen Idealkessel zu betrachtende Probenvorlaufgefäß, derart bestimmt ist, daß zu jedem beliebigen Zeitpunkt der Übernahme des Volumens V die für dieses Volumen repräsentative Konzentration im Anlieferungsbehälter im Probenvorlaufgefäß abgebildet ist.

3. Verfahren nach dem Oberbegriff des Anspruchs 1, dadurch gekennzeichnet, daß in dem nachfolgenden zweiten Zeitabschnitt ein Volumenstrom $Q_v(t)$ aus der Förderleitung in das Probenvorlaufgefäß überführt wird, der über ein festes Teilungsverhältnis, das demjenigen des ersten Zeitabschnittes entspricht, aus dem Volumenstrom Q(t) gewonnen wird, daß das Volumen des Probenvorlaufgefäßes V*(V) stetig und abhängig vom überführten Volumen

$$V(t) = \int_0^t Q(t)dt$$

nach einer Vergrößerungsfunktion M(V) vergrößerbar ist, daß ein Teilvolumen im Probenvorlaufgefäß, das dem neu hinzukommenden Anteil Platz macht, das Probenvorlaufgefäß mit dessen mittlerer Konzentration fortlaufend verläußt, und daß die Vergrößerungsfunktion M(V) derart bestimmt ist, daß hinsichtlich der Konzentration im Probenvorlaufgefäß die Wirkung nach dem Kennzeichen des Anspruchs 1 eintritt.

4. Verfahren nach Anspruch 1 oder 2, dadurch gekennzeichnet, daß das Teilungsverhältnis C(V) über eine Steuerung des Zulaufes des Probenvorlaufgefäßes einstellbar ist.

5. Verfahren nach Anspruch 1 oder 2, dadurch gekennzeichnet, daß das Teilungsverhältnis C(V) über eine Steuerung des Ablaufes des Probenvorlaufgefäß einstellbar ist.

6. Verfahren nach einem der Ansprüche 1 bis 5, dadurch gekennzeichnet, daß die Teilungsfunction C(V) bzw. die Vergrößerungsfunktion M(V) durch impulsweise Ansteuerung der letztere realisierenden Vorkehrungen dargestellt wird.

7. Verfahren nach Anspruch 6, dadurch gekennzeichnet, daß entweder die Impulsfrequenz f bei konstanter Impulsbreite $d_o$ oder die Impulsbreite d bei konstanter Impulsfrequenz $f_o$ verändert wird.

8. Verfahren nach einem Ansprüche 1 bis 7, dadurch gekennzeichnet, daß die Messung des bis zu einem bestimmten Zeitpunkt t überführten Volumens V(t) in einem, einem Luftabscheider vorgeschalteten, Abschnitt der Förderleitung erfolgt.

9. Verfahren nach einem der Ansprüche 1 bis 7, dadurch gekennzeichnet, daß die Messung des bis zu einem bestimmten Zeitpunkt t überführten Volumens V(t) in einem, einem Luftabscheider nachgeschalteten, Abschnitt der Förderleitung erfolgt.

**Revendications**

1. Procédé d'obtention d'échantillons liquides, en particulier d'échantillons de lait, à partir d'une conduite de transport allant d'un récipient de livraison à un récipient de collecte,

selon lequel, pendant le transport d'un volume de liquide à contrôler, un volume proportionnel au volume total est dérivé, receuilli et mélangé dans un récipient de prétraitement d'échantillon et, à la fin du transport, est séparé en un volume d'échantillon indépendant du volume total et en un volume résiduel, et le volume d'échantillon et le volume résiduel sont transférés respectivement dans un récipient d'échantillonnage et dans le récipient de collecte,

et selon lequel la dérivation dans le récipient de prétraitement d'echantillon a lieu en deux périodes de temps, pendant lesquelles des rapports de partage différents sont réalisés, un volume de base, obtenu avec un rapport de partage constant à partir d'une partie du volume total à transporter, étant transféré à la fin de la première période de temps, dans le récipient de prétraitement d'échantillon, caractérisé en ce que:

pendant la seconde période de temps qui suit, si un volume partiel ultérieur se trouve encore à prendre en charge, un écoulement volumique déterminé selon une fonction de partage C(V) est déversé en continu dans le volume de base, qui est maintenu constant, et mélangé avec lui, en ce qu'un écoulement volumique correspondant quantitativement à l'écoulement de liquide apporté est prélevé en continu dans le du volume de base, et en ce que la fonction de partage C(V), à chaque moment du transfert du volume liquide à contrôler, est déterminée à partir du volume total transporté jusqu'à ce moment et de la fonction de partage de temps de séjour pour le récipient de prétraitement d'èchantillon considéré comme un récipient continuellement agité, de telle façon que, quel que soit le moment du prélèvement d'un volume V, la concentration représentative pour ce volume dans le récipient de livraison est représentée dans le récipient de transport d'échantillon.

2. Procédé selon la revendication 1, caractérisé en ce que:

pendant la deuxième période de temps, un écoulement volumique $Q_v(V)$ est transféré dans le récipient de prétraitement d'échantillon, avec des rapports de partage C(V) variables, diminuant continuellement, dont la valeur initiale correspond à la valeur de la première période de temps et qui sont dépendants des volumes transportés jusqu'à l'instant t selon l'équation:

$$V(t) = \int_o^t Q(t)dt$$

la fonction de partage C(V) dépendante de la fonction de distribution des temps de séjour $W(t)=1-exp(-t/r)$ pour le récipient de prétraitement d'échantillon, considéré comme un récipient fictif agité continuellement, étant déterminée d'une telle manière qu'à n'importe quel moment du prélèvement d'un volume V, la concentration représentative pour ce volume dans le récipient de livraison est représentée dans le récipient de prise d'échantillon.

3. Procédé selon l'énonce de la revendication 1, caractérise en ce que:

pendant la deuxième période de temps qui suit, un écoulement volumique $Q_v(t)$ est transféré à partir de la conduite de transport dans le récipient de prétraitement d'échantillon, cet écoulement étant obtenu avec un rapport de partage fixe, qui correspond à chaque fois à la première périod de temps, à partir de l'écoulement volumique Q(t), en ce que le volume de récipient de prétraitement d'échantillon V*(V) est susceptible d'être augmenté de façon continue en fonction du volume transféré par la formule:

$$V(t) = \int_o^t Q(t)dt$$

selon une fonction d'accroissement M(V), en ce qu'un volume partiel, qui fait place à la nouvelle partie afférente, quittte le récipient de prétraitement d'échantillon avec cette concentration moyenne et en ce que la fonction d'accroissement M(V) est déterminée de telle sorte que la concentration dans le récipient de prétraitement d'enchantillon produit on effet selon la caractéristique de la revendication 1.

4. Procédé selon la revendication 1 ou 2, caractérisé en ce que:

le rapport de partage C(V) est réglable par l'intermédiaire d'une commande de remplissage du récipient de prétraitement d'échantillon.

5. Procédé selon la revendication 2, caractérisé en ce que:

la rapport de partage C(V) est réglable par une commande de l'évacuation du récipient de prétraitement d'échantillon.

6. Procédé selon l'une quelconque des revendications 1 à 4, caractérisé en ce que:

la fonction de partage C(V) ou la fonction d'accroissement M(V) est représentée par la commande par impulsions des dernières opérations en cours de réalisation.

7. Procédé selon la revendication 6, caractérisé en ce que:

soit l'impulsion f est modifiée pour une largeur d'impulsion constante $d_o$, soit la largeur d'impulsion d est modifiée pour une impulsion de fréquence constante $f_o$.

8. Procédé selon l'une quelconque des revendications 1 à 7, caractérisé en ce que:

la mesure du volume transporté V(t) jusqu'à un moment détérminé t est réalisée dans une section raccordée en amont à un séparateur d'air, de la conduite de transport.

9. Procédé selon l'une quelconque des revendications 1 à 7, caractérisé en ce que:

la mesure du volume transporté V(t) jusqu'à un moment détérminé t est réalisée dans une section, raccordée en aval à un séparateur d'air, de la conduite de transport.

**Claims**

1. Process for obtaining liquid samples, particularly milk samples, from a conveyor pipe leading from the supply vessel to a collection tank, wherein during the transfer of each liquid volume to be tested a volume proportionate to the total volume is branched off, collected and mixed in a preliminary sample vessel and after the termination of the transfer divided into a sample volume and a residual volume independent from the total volume, the sample volume being discharged into a sample vessel and the residual volume being discharged into a collection tank, and wherein the branching off into the preliminary sample vessel is effected in two periods of time, in each of which there are effective different division ratios, wherein at the end of the first period of time there is transferred to the preliminary sample vessel a basic volume obtained, on the basis of a constant division ratio, from one portion of the total volume to be transferred, characterized in that

in the subsequent second period of time, if there is still a further partial volume waiting for transfer, a volume stream to the basic volume, determined according to a division function C(V), which is maintained constant, is branched off continuously and mixed therewith, that a volume stream quantitatively corresponding to the added volume stream is continuously discharged from the basic volume, and that the division function C(V) at each point of time of the transfer of the liquid volume to be tested obtained from the total volume and transferred until that point of time and the assigned residence time division function for the sample vessel, considered as preliminary sample vessel, continuously operated as stirring vessel is determined such that at each optional point of time of the transfer of volume V the concentration representative for this volume is displayed in the supply vessel in the preliminary sample vessel.

2. Process according to claim 1, characterized in that in the second period of time with variable continuously reducing division ratios C(V), the initial value of which corresponds to that of the first period of time and which depend on the volume

$$V(t) = \int_0^t Q(t)dt$$

transferred until the point of time t, a volume stream $Q_v(V)$ is transferred to the preliminary sample vessel, the division function C(V), depending on the residence time division function $W(t)=1-\exp(-t/\tau)$ for the preliminary sample vessel considered approximately as continuously operated ideal vessel, being determined in a manner that each arbitrary point of time of the transfer of volume V the concentration representative for this volume is displayed in the supply vessel in the preliminary sample vessel.

3. Process according to the preamble of claim 1, characterized in that

in the subsequent second period of time a volume stream $Q_v(t)$ is transferred from the transport conduit to the preliminary sample vessel, said stream being obtained from the volume stream Q(t) via a fixed division ratio corresponding to that of the first time period, that the volume of the preliminary sample vessel V*(V) can be increased continuously and in dependency of the transferred volume

$$V(t) = \int_0^t Q(t)dt$$

according to an enlargement function M(V), that a partial volume in the preliminary sample vessel which gives space to the newly added portion, continuously leaves the preliminary sample vessel with its middle concentration, and that the enlargement function M(V) is determined thus that with regard to the concentration in the preliminary sample vessel the effect according to the characterizing part of claim 1 occurs.

4. Process according to claims 1 or 2, characterized in that the division ratio C(V) can be adjusted via a control of the feed of the preliminary sample vessel.

5. Process according to claims 1 or 2, characterized in that the division ratio C(V) can be adjusted via a control of the discharge of the preliminary sample vessel.

6. Process according to any one of claims 1 to 5, characterized in that

the division function C(V) or the enlargement function M(V) is shown by pulse control of the provisions realizing the latter.

7. Process according to claim 6, characterized in that either the pulse frequency f is modified with constant pulse width $d_o$ or the pulse width d is modified with constant pulse frequency $f_o$.

8. Process according to any one of claims 1 to 7, characterized in that

the measurement of the volume V(t) to be transferred until a specific point of time t is performed in a section of the transport conduit disposed ahead of an air separator.

9. Process according to any of claims 1 to 7, characterized in that

the measurement of the volume V(t) to be transferred until a specific point of time t is performed in a section of the transport conduit disposed subsequent the air separator.

Fig. 1

Fig.2

Fig.3

Fig. 4